# EUROPEAN PATENT APPLICATION

(11) **EP 3 885 370 A1**
(43) Date of publication of application: **29.09.2021**
(21) Application number: 20165089.2
(22) Date of filing: 24.03.2020
(51) Int. Cl.: C08B 31/10, A01N 25/10, C08B 33/04

(54) **BIOCOMPATIBLE CARRIER FORMULATION FOR APPLICATIONS IN PLANT PROTECTION AND PLANT GROWTH STIMULATION OR IN COSMETICS AND PERSONAL CARE ON THE BASIS OF MODIFIED STARCH**

(71) Applicant: Amynova Polymers GmbH, 04442 Zwenkau (DE)
(72) Inventor: SACK, Bernhard, 04442 Zwenkau (DE); MÜLLER, Steffen, 04442 Zwenkau (DE); KÖHLING, Sebastian, 04347 Leipzig (DE)
(74) Representative: Beyer, Andreas

(57) **Abstract**

A composition comprising hydroxyalkyl starch having an amylose content of at least 30 wt% and the weight average molar mass in the range of about 10⁵ - 10⁸ g/mol, a salt which is selected from the group consisting of an alkaline metal salt and/or alkaline earth metal salt, and water.

The preferably viscous formulation is especially suitable in the field of agriculture, horticulture and forestry to increase plant production efficiency by forming a film which reduces the wash-off of plant protection products, stimulants and fertilizers, or reduces erosion by soil bonding and dust formation in seed coating and animal husbandry.

Furthermore, the formulation is applicable as replacer of synthetic polymers in cosmetics and personal care products.

## Description

### FIELD OF THE INVENTION

The invention relates to an aqueous composition, or a formulation, comprising a hydroxyalkyl starch, a film obtained from this composition on a seed, a soil, a plant, or a part of a plant, or an animal feedstuff, comprising a coating obtainable from the composition, the use of the composition in agriculture and cosmetics, and a cosmetic comprising the composition.

### BACKGROUND OF THE INVENTION

In general, starch is a very versatile natural polysaccharide depending on origin, granule structure, concomitant substances, amylose/amylopectin ratio and molar mass distribution. Therefore, native and modified starch is used in food, beverages and feed, fermentation processes, paper and corrugated board production, in cosmetics and the pharmaceutical industry etc. One important parameter in determining the properties of modified starch is the content of amylose. The majority of the starch containing fruits, plants and vegetables contain less than 30 % of amylose. The term high amylose starch (HAS) is not clearly defined, and we want to propose that HAS is related to an amylose content higher than 30% of the dry substance.

Modified HAS is known as food additive to improve the gel forming ability, freeze-thaw-stability and thickening properties. (W. Vorwerg, J. Dijksterhuis, J. Borghuis, S. Radosta and A. Kröger, Starch 2004, 56, 297-306; I. A. Wolff, H. A. Davis, J. E. Cluskey, L. J. Gundrum, and C. E. Rist, Industrial & Engineering Chemistry 1951, 43(4), 915-919)

Most of the recently used polymers in agriculture are from synthetic or natural origin. (F. Puoci, F. lemma, U. G. Spizzirri, G. Cirillo, M. Curcio and N. Picci. American Journal of Agricultural and Biological Sciences 2008, 3, 299-314).

Although some of these polymers are of natural origin, e.g. guar gum, alginate, pectin, chitosan, polyhydroxy butyrate (PHB) etc., they can be only isolated to a limited extent and in complex processes, causing limited availability and relatively high prices of the resulting products. Due to the fact, that a more efficient and sustainable agriculture is a global challenge, optimized starch-based products can represent great benefits in worldwide plant production. On the one hand, starch can be found in many plants, vegetables and fruits as storage polysaccharide in high quantity, on the other hand, the extraction is a standard process with an annual production of 88 million tons in 2018. (https://www.imarcgroup.com/native-starch-market). That means comparatively low prices for starch, which allows for an attractive source biopolymer for many different product developments. Furthermore, starch is biodegradable and biocompatible.

### Leaf coating:

Premature wash-off of pesticides or fertilizers from leaves caused by rain, irrigation or other means strongly reduces their efficiency and leads to complex environmental problems in soil and to unwanted transfer of chemicals into the ground water. Most of the active ingredients in plant protection products are of very small amounts or insoluble in water. Therefore, the main part of these formulations are emulsifiers or surfactants to create a sprayable aqueous dispersion. On the other hand, these chemicals enhance the wash-off effect on leaves during rainfalls and irrigation.

Commonly used sticking agents are based on surfactant or mineral oils to reduce the surface tension for better spreading or formation of oily films on leaves and soil. As a result, the natural cuticular wax layer on leaves is removed or partially dissolved which could induce plant stress. The leave wax layer naturally shields plants against dehydration and surface wetting. If the layer is affected by chemicals, the plant reacts initially with wilting leaves and over long term with slower growth and decreased crop yield.

A solid formulation of dispersible starch together with biological active substances and additional surfactants and adjuvants is described by U.S. Pat. No. 2003/0109384. The patent describes a solid formulation that can be dispersed in water and is composed of modified starch, e.g. esters or ethers, a biological active agricultural material, e.g. herbicides, insecticides and fungicides and optionally a surfactant or adjuvant.

### Soil coating:

Preventing erosion of soil especially in agricultural landscapes is one of the main challenges in crop cultivation. The loss of material like organic substances, seeds or fertilizers caused by wind and water reduces the fertility of the soil and crop yield to a considerable extent. One solution to prevent displacement, drift and leaching of soil can be the treatment of the soil surface with polymeric substances of preferably natural or synthetic origin. The bonded soil particles are more resistant to the mechanical impact of rain, flowing water and wind. A mixture comprising mulch and a cover composition including bentonite clay and cellulosic water dispersible polymer or starch is presented in patent US 9,771,516.

In patent U.S. Pat. No. 5,125,770, a mixture of pregelatinized starch and a surfactant, used in significant amounts, is described for stabilising soil and reduction of erosion.

Referring to patent U.S. Pat. No. 2,957,834, the soil top layer should be stabilized by applying a cold-water swelling starch solution containing formaldehyde as cross-linker which forms a synthetic resin by addition of urea, melamine, phenols, dicyanodiamide, and acetone as further additives.

### Seed coating:

Seeds are often coated or pelleted for protection from mechanical or drought stress, bacterial or fungal damage, and from insects, birds and rodents. Another important aspect of seed coating is the support of seeds during germination and the early stage of growth with coatings including pesticides, fertilisers and bio stimulants. Abrasion of seed coatings during the sawing process can induce serious environmental contamination, especially when fungicides, insecticides or bactericides are used. Therefore, one of the major challenges in seed coating is the reduction of dust formation. It seems reasonable to use polymers either as a final or as an intermediate coating during seed preparation. Ideally, this type of polymer should be readily biodegradable and additionally having one of the desired protective or growth stimulating effects.

WO 99/57959 discloses a film coated seed which is formed by film coating a seed with a starch-based, e.g., polysaccharide, water-dispersible or water-soluble polymer. The polysaccharide polymer is preferably produced by jet cooking the starch. One or more beneficial additives can be included in the film coating, including, for example only, adhesives or binders; plasticizers; colorants or dyes; hydrophobic and/or hydrophilic materials; insecticides, fungicides or herbicides; bacterial inoculants; nutrients; and plant growth regulators. The slurry treatment typically adds about 0.1 to 0.5 % by weight and film coating typically adds about 0.5 to 2 % weight to the seed.

A polymer blend seeds coating formulation formed from crosslinked amylose or amylopectin was presented in US 2019/0150354 A1.
Whereby the other components, a second binder, are synthetic polymers or modified guar. It should be mentioned that the isolation process of amylose is very special and challenging.

In patent WO 99/51210, coating comprising a modified starch and a plasticizer claimed.

Another form of starch is used as superabsorbent fertilizer and micronutrient.

US 7,423,106 discloses A superabsorbent polymer product for use in agricultural applications, the polymer product made by the following steps:
providing grafting reactants and a starch; graft polymerizing the grafting reactants onto the starch to form a starch graft copolymer; saponifying the starch graft copolymer;
precipitating the saponified starch graft copolymer; and granularizing the precipitated starch graft copolymer so as to form particles having a density that is between about 30 pounds per cubic foot and about 35 pounds per cubic foot.

However, the starch-based polymer particles could have limited penetration ability on soil compared to starch dispersions or solutions. In addition, the synthetic copolymer which is grafted onto the starch backbone reduces the biodegradability and can act possibly toxic after starch degradation.

### Root coating:

In US 7,607,259 a superabsorbent polymer hydrogel is presented as "root dip" for improved storing, transport or planting of exposed roots. The main component, a cross linked starch-based copolymer grafted with acrylamide or acrylic acid formulated as potassium salt can be used as standalone coating, or in combination with at least one agricultural additive, like e.g. fertilizer, pesticide, herbicide, fungicide, and growth regulators. Due to the used synthetic acrylamide/acrylic acid for grafting, the starch formulation is environmentally problematic and has limited biodegradability and biocompatibility.

### Dust control:

Airborne dust is a global phenomenon and can create serious environmental, health and safety problems, e.g. in mining facilities, agriculture, traffic, industry and construction. Dust particles can carry micro fauna like bacteria and viruses over thousands of kilometres, whereas the dust distribution is mainly dependent on the size of the particles. Therefore, bonding of fine airborne dust particles in an effective and environmentally friendly way is the requirement.

In patent CA 2868855, a polymer dispersion was produced by emulsion polymerisation on the base of styrene or modified styrene, acrylic- or methacrylic acid ester, acrylo- or methyacrylonitril, an ethylenically unsaturated monomer and native, modified or degraded modified starch. It can be assumed that the biodegradability is affected depending on the formulation of the synthetic monomers.

Similar formulations including hydrolysates of starch acrylonitrile grafted copolymers, partially neutralized starch-acrylic acid or starch-polyvinyl alcohol grafted copolymers were presented in US 6,090,875.

### Cosmetic and personal care:

Polymers are widely used in cosmetic and personal care application. The spectrum of usage and types of polymers is broad. Usually, polymers of synthetic or silicon origin are applied as thickening agents, emulsifiers, barrier formers, film formers, wetting agents and for aesthetic reasons. Especially microplastics and soluble plastic made from polyacrylates (preferably carbomer) are frequently used in cosmetics and are problematic for environmental reasons. There is a high demand for natural and readily biodegradable and biocompatible alternatives for polymers from synthetic or petrochemical origin in cosmetics and personal care products.

In patent EP 1 389 459, the inventors described a hydrocolloidal formulation from hydroxypropyl starch phosphate to improve the availability of active substances in cosmetic and dermatological preparations. The hydroxypropyl starch is crosslinked by phosphates and as 0.1 to 10 wt-% included. The colloidal formulation can be used alone or in combination with other hydrocolloids and should be especially suitable for skin care applications.

In U.S. Pat. No. 7,361,363, similarly, a formulation containing a hydroxypropyl starch crosslinked by phosphate bridges with high glycerine content, potentially fatty alcohol and an additional surfactant is claimed as "silky sensory feel" cosmetic formulation.

In summary, for better processing of starch, e.g. for coating purposes, highly dissolved starches or starch products would be beneficial.

Starches which are called dissolved starches are often gelatinized starches which are composed of highly swollen starch granule fragments, and of microgels and partly dissolved starch polysaccharides. Dissolved starch polysaccharides could be present as more or less minor fraction. Therefore these aqueous starch dispersions could show the disadvantage of phase separation and even retrogradation with gel formation. Best dissolution states of starches are obtained by jet cooking at temperatures > 120°C or at pH > 12 or by chemical modification.

In summary, known chemically modified starches do not have the beneficial characteristics of water solubility, appropriate film formation without plasticizer and have limited biodegradability. The preparation of aqueous dispersions by additional equipment is also necessary in situ. Aqueous dispersions can be mixed with various plant protecting and stimulating substances, but the application and storage time could be also limited.

The replacement of synthetic polymers in commercially aqueous plant protecting and growth stimulating dispersions might be possible with added investment (dispersion preparation), effort and usage risk because of limited storage stability.

### OBJECT OF THE INVENTION

The object of the invention was the development of an aqueous long-term stable composition which is suitable for formation of a film coating, particularly for coating of leaves, seed, roots or soil, or other matter, or for modifying the properties of cosmetics. Preferably, the composition should be biodegradable.

### SUMMARY OF THE INVENTION

The invention provides a composition comprising
a hydroxyalkyl starch or a hydroxyalkyl starch fraction, having an amylose content of at least 30 wt% and a weight average molar mass in the range of about 10⁵ g/mol to about 10⁸ g/mol, preferably in the range of about 10⁵ g/mol to about 4·10⁷ g/mol,
Furthermore, the formulation contains salt, which is selected from the group consisting of an alkaline metal salt and/or alkaline earth metal salt, and water.

The composition, in its general or in one or more specific embodiments, has one or more of the following advantages:
- biodegradable
- ready to use
- easy to handle
- based on modified starch with high molar mass,
- mainly neutral substituents,
- compatibility with a wide variety of plant protection compounds and cosmetic ingredients in aqueous systems
- can be prepared as aqueous viscoelastic formulation
- provides long-term stability for more than several weeks
- has one or more of the following functional properties: thickening, viscosity regulation, film forming, moisturizing, barrier forming, wetting, sticking, delayed delivery of added chemical and biological compounds.

Moreover, the composition, in its general or in one or more specific embodiments, has one or more of the following advantages:
Hydroxyalkylation improves the applicability of HAS. Depending on the degree of substitution and distribution of substituents, a water-soluble starch, preferably soluble in water of 25°C in amounts as indicated later, and at the same time a high molecular starch product can be developed. We observed that Hydroxyalkylation is happening without any significantly molecular degradation.

In previous starch coating compositions of the prior art, the compositions of the prior art contain a significant portion of swollen starch particles. This formulation could lead to phase separation and change of viscosity. Another important issue is the processability of aqueous starch dispersions because of retrogradation. The present invention can overcome these issues be preferably providing the hydroxyalkyl starch or hydroxyalkyl starch fraction predominantly in solution.

The invention is directed to an aqueous formulation of high molecularly modified starch, which is particularly, but not only, beneficial in two main areas of applications. The first area is predominantly related to agriculture, orchards, seed treatment, vegetable gardening, ornamental plants, turf, landscape construction and protection or forest cultivation as well as feed. The second area has the focus on use as an ingredient of cosmetic and personal care products.

The modified starch exhibits compatibility or miscibility with a wide range of chemical and biological compounds, which can be incorporated in various concentrations. Furthermore, the liquid starch formulation is ready to use, easy to handle and further characterized among other by long-term stable viscosity, co-emulsifying, binding and adhesive properties, and film formation, also without plasticizer, by dehydration or drying, among other applications.

The aqueous liquid composition shows effects for instance in coating of seed, soil, plant or a part of a plant, or an animal feedstuff, as well as in the usage as carrier matrix, dust binding agent or prevention of dust formation. Beyond that, the modified starch composition is a biocompatible component, which is suitable as replacer of synthetic additives in plant protection, fertilizers and cosmetics or personal care products.

The Invention describes an aqueous, non-retrogradating viscous formulation with long-term stability, which is based on largely homogenously modified, highly molecular and high amylose starch ether with the advantages of thickening, viscosity regulation, binding, adhesive strength, co-emulsifying, film formation without any plasticizer, and compatibility with a lot of chemical and biological substances.

As starch is used as basis, the formulation has a renewable origin, is inexpensive and readily biodegradable and biocompatible. The described formulation shows surprising effects in agriculture applications even when applied on plant parts and soil in low concentration from 1-2L/ha. If the plant was treated with the mixture of aqueous starch ether formulation and hazardous plant protection products (PPP), the plants show significantly reduced stress reactions. The starch film reduces wash-off during rainfall and irrigation and provides a slow release for PPP or stimulating products included in the starch film when it is wetted again. Therefore, the PPP can be used more efficient and soil and ground water contamination can be avoided or reduced. As long-term effects, the plants are healthier, give higher crop yields and show in general more resilience to stress factors. Additionally, the formulation is suitable for soil application as bonding agent for the topsoil layer to reduce erosion caused by wind, rainfall and irrigation. As hydrophilic polymer, starch increases the water holding capacity of soil which results in higher water availability and reduced drought stress improves the uptake of nutrients by roots and increases microbial activity in the soil. In addition, the starch can act as nutrition for bacteria and fungi and therefore improves biological activity of the microbiome, which can improve plant growth and promotes biodegradation of synthetic chemicals applied to the soil. In further aspects, the aqueous starch ether formulation can be used as carrier matrix for fertilizers, growth regulators, biostimulators and plant strengthening agents. Further applications relate to plant growth stimulation in gardening and dust control in landscape preservation, orchards or animal husbandry. As one significant difference to the state of the art of modified starch, the presented aqueous high amylose starch ether formulation is storage stable for up to 24 months while maintaining the viscoelastic, castable properties and their ready to use and easy to handle character. In addition to the broad application profile in agriculture, gardening and landscape conservation, the modified HAS starch can meet special requirements in the area of cosmetics and personal care.

The new product is suitable to replace polymers from petrochemical or synthetic origin (microplastics, soluble plastics) due to thickening, viscosity regulation, co-emulsifying, moisturizing, film formation as well as the compatibility with many cosmetic or personal care ingredients.

The invention, using hydroxyalkyl starch or a hydroxyalkyl starch fraction as raw material for biodegradable polymers can be one of the solutions for the well-known problems in agriculture, like e.g. low pesticide and fertilizer efficiency, environmental contamination and the increasing demand for farming land. The hydroxyalkyl starch or hydroxyalkyl starch fraction can be produced inexpensively and is biocompatible and biodegradable. These polymers have a large potential and their use in agriculture could be substantially increased.

It has been found that for leave coating, soil coating, seed coating, root coating, dust control and personal care and cosmetics a starch with an amylose content as in the present invention is beneficial due to film forming ability

The hydroxyalkyl starch or hydroxyalkyl starch fraction in the composition of the invention is preferably non-granular and/or non-pregelatinized starch (fraction). By this measure the hydroxyalkyl starch or hydroxyalkyl starch fraction is even better water miscible, or even highly soluble, not only water dispersible/swellable.

The hydroxyalkyl starch or hydroxyalkyl starch fraction in the composition of the invention is able to form homogenous mixtures and therefore the film forming is significantly improved.

In order to form homogeneous layers on plants, roots or soil, no additives or additional polymers are necessary, like e.g. softeners, expensive natural polymers (e.g. guar gum) or even synthetic polymers like polyvinyl acetate or polyvinyl alcohol. So, the composition of the invention preferably does not contain such additives or additional polymers.

Preferably the hydroxyalkyl starch or hydroxyalkyl starch fraction in the invention is not further grafted with non-glucose monomers.

Preferably the hydroxyalkyl starch or hydroxyalkyl starch fraction in the invention is not further grafted with synthetic monomers, like e.g. acrylic acid, acrylamide, methacrylic acid, acrylonitrile etc., by radical polymerisation. By such synthetic polymer, biocompatibility and biodegradability would be reduced.

The invention provides a long term applicability, as a castable, viscoelastic, homogenous mixture and/or storage stable composition.

The properties of the modified starch can be further adjusted by the amylose/amylopectin content, average molecular weight, cross-linking, additional substituents (such as ether and ester) and the degree of substitution.

In the field of cosmetics, especially the long-term viscoelastic properties of the starch formulation are of high importance.

The invention allows the application of a film forming polymer dispersion with incorporated plant protection products, which is a solution to generate layers on leaves reducing wash-off of biological or chemical agents with the additional advantage of their slow release. Therefore, plant protection products can be used more efficiently, which could reduce the required amount and possibly reduces environmental contamination in the case of chemical protection agents.

The application possibilities are broad. Even though examples focus on the suitability for coating of seeds, soil, plants or plant parts, particularly in order to be used as carrier matrix for agrochemicals, biological substances, and for cosmetic and personal care ingredients, this does not limit the fields of application.

### DETAILED DESCRIPTION

If in this description numbers or terms are indicated with the word about, this encompasses the exact value or term. For example, an indication of "about 1" encompasses exactly 1 and the indication "about 1" may be limited to "1".

If in this description only hydroxyalkyl starch is mentioned, this term also encompasses a hydroxyalkyl starch fraction, if not otherwise stated.

The composition is an aqueous formulation.

The salt is at least partially, preferably completely, dissolved in the water.

Amylose content is preferably related to the total mass of the hydroxyalkyl starch or hydroxyalkyl starch fraction, preferably on a dry basis.

Amylose content may more preferably be determined in the starting starch or starting starch fraction, i.e. the starch or starch fraction before functionalization with hydroxyalkyl groups, preferably on a dry basis. It has however been turned out that this value does not differ from the amylose content related to the total mass of the hydroxyalkyl starch or hydroxyalkyl starch fraction, preferably on a dry basis.

The weight average molar mass is related to the hydroxyalkyl starch or hydroxyalkyl starch fraction, i.e. the starch or starch fraction after functionalization with hydroxyalkyl groups.

An upper limit of amylose may be 99 wt%, or 95 wt%, or 90 wt%.

The weight average molar mass may depend on the amylose content. In case of a high amylose content, such as 99 wt%, the substance is a starch fraction mainly composed of amylose. The higher the amylose content, the lower the molar mass preferably is, until to a lower limit of a weight average molar mass is about 10⁵ g/mol, which is preferably the weight average molar mass of pure or isolated hydroxyalkylated amylose

If the amylopectin content increases, the weight average molar mass preferably increases too, preferably until an upper limit of 10⁸ g/mol, or 4.10⁷ g/mol, is reached. The starch is then a hydroxyalkylated high amylose starch.

Starches with amylose content of at least 30 wt% are known from the prior art and commercially available. Every available starch with amylose content of at least 30 wt% is suitable by specific optimization of etherification process in dependence on the kind of starch, because of variation of amylose content, granule structure and concomitants.

Another upper limit of weight average molar mass, which could be combined with any of indicated lower limits, is 5·10⁷ g/mol.

In the composition the hydroxyalkyl starch or hydroxyalkyl starch fraction may be present predominantly dissolved. Dissolved means preferably molecularly dispersed. Preferably, more than 50 wt% of the hydroxyalkyl starch or hydroxyalkyl starch fraction, related to the total mass of the hydroxyalkyl starch or hydroxyalkyl starch fraction in the composition of the invention, preferably related to dry matter of the starch/-fraction, is dissolved, more preferably at least 60 wt%, at least 70 wt%, at least 80 wt%, at least 90 wt%, or at least 95 wt%. In the composition some of the hydroxyalkyl starch or hydroxyalkyl starch fraction, which may be the remainder of the starch or starch fraction which is not completely dissolved, may be present in colloidal form (such as microgels) and/or in swollen particle form, preferably in colloidal form.

The formulation is a liquid formulation and hereinafter also called a liquid formulation. The liquid formulation is completely water miscible at any ratio.

The salt may further act as liquid fertilizer or pH-buffering substance or both.

The liquid formulation is capable to form a film after (partial) drying. It has been found that foils and products obtained or obtainable by the composition have excellent film forming properties.

Preferably, the composition and its formed films which are obtained from the composition do not incorporate any non-biodegradable chemical additives or plasticizer.

Preferably, the composition and the formed films which are obtained from the composition are completely biodegradable.

Preferably, the composition has co-emulsifying properties. Miscibility can be obtained with fatty acids without surfactant addition at least up to 15% vol-% of aqueous formulation. The chemical composition of fatty acids influences their miscibility with the aqueous formulation.

The liquid formulation can be used after mixing with an active substance, also called active ingredient, which may be applied to a plant, plant part, seed or soil as well as cosmetics and plant care products.

The liquid formulation can be used together with an active substance, i.e. when the active substance is comprised in the composition.

The liquid formulation is preferably for use in the areas: agriculture (such as plant production, turf, orchards, plantations, specialty cultures, flowers), forestry, greenhouses, home gardens, nurseries, indoor farming, animal husbandry and cosmetics and personal care.

The composition is especially suitable for seed coating, seed pelleting, soil coating, dust reduction (exemplary applications are feed, feed lots, orchids, mining), anti-leaching, plant coating, leaf coating, root coating, fruit and vegetable coating, as carrier matrix for plant protection products, plant growth stimulators and as standalone bio stimulant. In general, the formulation is applicable for anything what needs to be coated and granulated.

Seed pelleting in a preferred meaning means a process in which small or irregularly shaped seeds are coated with an inert material to make them round and uniform. Seed coating in a preferred meaning means a thicker form of covering of seed and may contain fertiliser, growth promoters and or seed treatment as well as an inert carrier and a polymer outer shell.

In the invention, advantageous results are attained if the basic starch is derived from the group of high amylose starches and if its amylose content is not less than 30%, preferably at least 50%. A very low proportion of hard-to-dissolve components are obtained by a hydroxyalkylation, preferably under mild conditions.

Following the hydroxy alkylation, the starch derivative may be obtained amorphous, non-degraded, molecularly and/or colloidal disperse.

The composition of the invention may be obtained as a viscoelastic aqueous composition. The composition preferably has long-term stability, whereas the starch in the composition is non-degraded and exhibits an excellent and long-lasting solution state. The composition may be further processed by known processes such as spraying, spreading and casting.

The liquid formulation can be easily mixed with water at any ratio, i.e. without phase separation. In other words, the liquid formulation is completely or nearly completely water soluble at any ratio.

The viscosity of the liquid formulation depends on the concentration of modified starch and the content of ingredients and decreases with higher water content. The dynamic viscosity has a range from 0.1- 2.5 Pa·s (at 25°C and shear rate of 30 s⁻¹). A measurement method is given in the examples section.

The liquid formulation has excellent film forming properties even without the addition of plasticizer like, e.g. glycerol, sorbitol, polyethylene glycol or glucose. The liquid formulation can be used as aqueous component in mixtures, or pure.

The composition is reverse water soluble. That means the composition may be dried and rehydrated for the purpose of the preparation of an aqueous solution.

The composition is able to act as delayed-release matrix. In the composition, active substances, like agrochemicals or plant strengthening agents or cosmetic ingredients can be included and released over time.

The liquid formulation, particularly with low salt content, can be used in cosmetic and personal care products. The composition of the invention, or a film obtainable from the composition, may be used as or for a cosmetic or personal care application, preferably as agent for thickening, co-emulsifying, film forming, wetting, sticking, protective barrier and viscosity regulator.

Etherification is the preferred starch modification of this invention. Basis methods are described by Rutenberg, M.,W. and Solarek, D.: Starch derivatives, production and uses. Book: Starch: Chemistry and Technology (Ed. Whister, L.) p. 343-349 (1984) and in the publication of Roth, W.B., Mehltretter, C.L.: Some properties of hydroxy propylated amylomaize starch films. Food Technol. Vol.21, p.72-74 (1967). The hydroxy alkylated, preferably hydroxy propylated, starch, prepared according to these methods, can be dispersed in water at room temperature or by jet-cooking.

Film properties of undegraded hydroxyalkylated starch, which is dissolved in water in the composition, are affected by amylose content and the type of starch used. Whereas high values give more flexible films with higher mechanical strength. Another additional factor responsible for good film forming and long-term stable viscosity are the kind of substituent, the optimal MS value as well as the distribution of substituents along the carbohydrate backbone, where higher homogeneity results in better film formation and longer stability. In the invention, it is not necessary to use plasticizers or other synthetic or biopolymers additives in order to support film formation, or to support film formation with reduced brittleness, as it is the case when the amylose content is lower.

The present invention overcomes problems of unmodified starch. In the case of film forming, an unmodified starch has turned out to be only applicable under the precondition of complete dissolution of starch by pressure cooking at a temperature higher than 110°C and processing of the starch solution at relatively high temperatures because of retrogradation. Starch gelatinization obtained by cooking starch in water at elevated temperatures results in heterogeneous aqueous dispersion with only partly dissolved amounts of starch and a relative high proportion of supramolecular structures.

The present invention allows a degree of substitution with largely homogeneous distribution of substituents in the linear polysaccharide chains as well as in the out side chains of branched polysaccharides,. This chemical structure is beneficial for long term stable viscosity, to prevent retrogradation and phase separation in aqueous systems of modified starch products. The degree of substitution may be adjusted depending on starch type and amylose content. In this sense it is possible to develop the basis of an aqueous modified starch formulation with long-term stability of rheological properties. A specific determination method is given in the examples section.
So, the invention provides a composition, wherein the hydroxyalkyl starch or hydroxyalkyl starch fraction is largely homogenously substituted.

Amylose content was determined amperometric. A specific method is recited in the examples section.

The alkyl moiety in the hydroxyalkyl starch or hydroxyalkyl starch fraction is preferably selected from methyl, ethyl, propyl (n-propyl or iso-propyl), butyl (n-butyl, iso-butyl or tert-butyl). A very suitable hydroxyalkyl starch or hydroxyalkyl starch fraction is hydroxypropyl starch or hydroxypropyl starch fraction, wherein propyl is preferably n-propyl.

In one embodiment of the composition, the content of the hydroxyalkyl starch or hydroxyalkyl starch fraction in the composition is about 5 to about 25 wt% (weight percent), related to the whole composition, preferably about 5 to 20 wt%, more preferably 7 to 15 wt% and still more preferably from 8 to 12 wt%.

In one embodiment of the composition the content of the salt is 0.01 to about 10 wt%, related to the whole composition, preferably 0.02 to about 10 wt%, more preferably 0.05 to about 10 wt%, or 0.05 to 8 wt%.

Further ranges of salt, preferably applied when the compostition is used in the field of agriculture, are about 2 to 10 wt%, preferably from 5 to 9 wt% and more preferably from 6 to 8 wt%.

Further ranges of salt, preferably applied when the compostition is used in cosmetics and personal care, are about 0.01 to 10 wt%, preferably from 0.01 to 5 wt% and more preferably from 0.01 to 2 wt%.

In one embodiment of the formulation, the modified starch has a degree of molar substitution (MS) of 0.05 to about 0.8, preferably about 0.2 to about 0.7 and more preferably from 0.3 to 0.6. The value is preferably adjusted to the amylose content of the starting starch or -fraction. A measurement method is given in the examples section. The MS is related to all substituted available hydroxy groups, particularly at positions C₂, C₃ and C₆ in the anhydro glucose units and is additionally related to hydroxy groups comprised in the substituent. So, the MS encompasses one or more further alkylene oxide moieties which are attached to a hydroxy group of a functional group (which is attached to a C₂, C₃ or C₆ in the anhydro glucose unit). MS reflects the average number of moles of alkylene oxide directly and indirectly attached to the anhydroglucose unit.

The degree of substitution (DS) is related to the introduced ether groups in the positions C₂, C₃ or C₆ of the anhydro glucose units only. DS reflects the number average of substituted hydroxyl groups in anhydroglucose units. So, DS does, in comparison to MS, not comprise indirectly attached alkylene oxide moieties.

In the case of homogeneous substitution of starch, the anhydro glucose unit bearing the statistical value of substituents according to the determined degree of substitution DS. In case of DS = 1 every anhydroglucose unit has one substituent. For DS = 0.5, every second glucose unit has one substituent, or in other words, 50% of the pyranose rings are substituted and 50% not. In case of heterogeneous substitution, some pyranose rings have more than one substituent (di-, tri- substituted). In case of DS = 0.5 after total hydrolysis of the starch polymer 50% (very homogeneous substitution) or more than 50% (the higher the value, the more inhomogenous the substitution) of unsubstituted glucose will be found as additional feature.

The value of the degree of substitution (DS) is preferably 20-90% of the value of the molar degree of substitution (MS). DS may be determined by the synthesis conditions, such as excess alkylene oxide, pH value, temperature, starch concentration, reaction time.
DS may be in the range of about 0.01 to about 0.72, about 0.04 to about 0.63, about 0.06 to about 0.54, about 0.025 to about 0.6, about 0.1 to about 0.5, about 0.15 to about 0.4, or about 0.1 to about 0.5.

The proportion of unsubstituted glucose units in the hydroxyalkyl starch or hydroxyalkyl starch fraction of the invention is preferably 1 - DS. The proportion of unsubstituted glucose units in the hydroxyalkyl starch or hydroxyalkyl starch fraction of the invention is preferably 0.4 to 0.975, 0.5 to 0.9, or 0.6 to 0.85. These values could be expressed as percentages, and also MS and DS, by multiplying with 100.

In one embodiment of the composition, the hydroxyalkyl starch or hydroxyalkyl starch fraction has a predominantly non-granular, amorphous structure. The term predominantly means that at least 95 wt% of the starch or starch fraction, related to the total mass of the hydroxyalkyl starch or hydroxyalkyl starch fraction in the composition of the invention, preferably based on dry matter of the starch/-fraction, has a non-granular, amorphous structure.

In one embodiment of the composition the salt has a chalcogen, or pnictogen comprising anion.

In one embodiment of the composition the anion is selected from phosphate, hydrogen phosphate, sulfate, hydrogen sulfate, or acetate.

In one embodiment of the composition the salt is selected from sodium dihydrogen phosphate, disodium hydrogen phosphate, sodium phosphate, sodium hydrogen sulfate, sodium sulfate, potassium dihydrogen phosphate, dipotassium hydrogen phosphate, potassium phosphate, potassium hydrogen sulfate, potassium sulfate, sodium acetate, potassium acetate, calcium acetate.

In one embodiment of the composition, the composition further comprises an ingredient selected from the group of terpenes, a humic substance, an agrochemical, a biostimulator, a plant strengthening agent, or a preserving substance, preferably a preserving substance with the ability to avoid microbial and fungal activity.

The amount of ingredient in the composition of the invention (liquid formulation) may be 0.001 - 10 wt% related to the whole composition, or 0.001 - 5 wt%, 0.001 - 2 wt%, 0.001 - 1 wt%, or 0.001 - 0.1 wt%.

In the invention, an agrochemical is preferably selected from a fertilizer, a plant protection product, or a plant growth regulator.

A plant protection product may be a pesticide, herbicide, insecticide, fungicide, bactericide, acaricide, nematicide or molluscicide.

Plant protection products, particularly single active ingredients or combined active ingredients (without limitation on specific amounts), which are suitable for the present invention are listed in the
"List of Authorised Plant Protection Products in Germany, with Information on Terminated Authorisations" (Date: January 2020) by the Bundesamt fur Verbraucherschutz und Lebensmittelsicherheit of the Federal Republic of Germany, which is incorporated by reference in its entirety.
This list is electronically available from: www.bvl.bund.de/infoppp (On the english page in the version of 28.02.2020 choose the link "concise list of plant protection products (Jan. 2020)) or www.bvl.bund.de/infopsm (on the german page in the version of 28.02.2020 choose the link "Übersichtsliste der zugelassenen Pflanzenschutzmittel in Deutschland mit Informationen über beendete Zulassungen (Januar 2020)).

Particularly suitable herbicides are shown in following table:

**(HRAC = Herbicide Resistance Action Committee)**

| **HRAC Group** | **Mode of action** | **Family (examples)** | **Active agent (examples)** |
|---|---|---|---|
| **A** | Inhibition of Acetyl CoA Carboxylase (ACCase) | Aryloxyphenoxy-propionate (FOP) | Clodinafop-propargyl |
| | | | Haloxyfop-R-methyl |
| | | | Propaquizafop |
| | | | Quizalofop-P-ethyl |
| | | Cyclohexanedione (DIM) | Clethodim |
| | | | Cycloxydim |
| | | Phenylpyrazoline (DEN) | Pinoxaden |
| **B** | Inhibition of Acetolactat Synthase (ALS) | Sulfonyl urea | |
| | | | Foramsulfuron |
| | | | Iodosulfuron |
| | | | Mesosulfuron |
| | | | Metsulfuron-methyl |
| | | | Nicosulfuron |
| | | | Rimsulfuron |
| | | Imidazolinone | Imazamox |
| | | Triazolopyrimidine | Penoxulam |
| | | | Pyroxsulam |
| | | Sulfonylaminocarbonyl -triazolinone | Propoxycarbazone -Na |
| **C 1** | Inhibition of Photosynthesis of Photosystem II | Triazine | Terbutylazin |
| | | Triazinone | Metamitron |
| | | | Metribuzin |
| | | Pyridazinone | Chloridazion |
| **C 2** | Inhibition of Photosynthesis of Photosystem II | Urea | Chlortoluron |
| **E** | Inhibition of Protoporphyrinogen Oxidase (PPO) | N-phenylphthalimide | Flumioxazin |
| **F 1** | Bleaching: Inhibition of Carotenoid Biosynthesis in Phytoene Desaturase Step (PDS) | Other | Beflubutamid |
| | | | Flurtamone, |
| | | | DFF |
| **F 2** | Bleaching: Inhibition of 4-Hydroxyphenyl-Pyruvate-Dioxygenase (4-HPPD) | Triketone | Mesotrione |
| | | Isoxazole | Isoxaflutole |
| **F 3** | Bleaching: Inhibition o Carotenoid Biosynthesis | Osoxazolidinone | Clomazone |
| | | Diphenylether | Aclonifen |
| **G** | Inhibition of Enolpyruvylshikimat-Phosphat-Synthase (EPSP Synthase) | Glycine | Glyphosat |
| **H** | Inhibition of Glutamin Synthetase | Phosphinic acid | Glufosinate (-Ammonium) |
| **K 1** | Inhibition of Microtubuli-formation | Dinitroaniline | Pendimethalin |
| | | Benzamide | Propyzamid |
| **K3** | Inhibition VLCFAs (Inhibitor of cell division) | Chloroacetamide | Metazachlor |
| | | | Metolachlor |
| | | | Pethoxamid |
| | | Acetamide | Napropamid |
| | | Oxyacetamide | Flufenacet |
| **N** | Inhibition Lipid Synthesis - non ACCase Inhibitor | Thiocarbamate | Prosulfocarb |

https://pflanzenschutzdienst.rp-giessen.de/ackerbau/pflanzenschutzempfehlungenackerbau/wintergetreide-allgemein/unkraut-und-ungraskontrolle/uebersicht-ueberherbizid-wirkstoffklassen/

A plant protection product may be a plant protection product of natural origin.

The fertilizer may be a synthetic fertilizer or of organic origin.

A composition comprising a terpene has preferably the ability to avoid microbial activity. The terpene is preferably a monoterpenoid, preferably geraniol. The content of terpene may be about 0.05 to 0.2 wt%, preferably about 0.05 - 0,15 wt% of the composition.

A biostimulator is preferably selected from a compound from the group consisting of amino acids, peptides, antioxidants or products from microbe fermentation and algae.

The composition may be further diluted in an aqueous solvent. The composition may be diluted together with a compound selected from a terpene, a humic substance, an agrochemical, a biostimulator or a plant strengthening agent.

In one embodiment, the composition does not contain a plasticizer.

In one embodiment, the composition does not contain a non-biodegradable additive.

In one embodiment, the composition is biodegradable, preferably fully (100%) biodegradable.

In one embodiment, the formulation comprises a preserving substance with the ability to avoid microbial and fungal activity. The substance is preferably from the group of medium or long-chain carboxylic acid and carboxylates, a plant oil, essential oil, sorbic acid and sorbates, benzoic acid and benzoates, chitosan, isothiazolinones and benzisothiazolinone, The content may be about 0.01 to 0.3 wt%, preferably about 0.01 - 0,15 wt% of the formulation.

In one embodiment, the hydroxyalkyl starch or hydroxyalkyl starch fraction is cross-linked. Cross linking may be used to regulate viscosity, which is particularly suitable for use in the field of cosmetic. Useful crosslinking agents are: low molecular weight aldehydes, dialdehydes, ketones, diketones, oxidative agents, like e.g. formaldehyde, glyoxal, glutaraldehyde, pyruvic acid; organic, multi-base acid chlorides and their derivatives, like e.g. pyruvic acid, glutaric acid, citric acid, adipic acid, malonic acid, malic acid, tartaric acid; Inorganic cross-linking reagents, inorganic multi-basic acids, alkaline-hypochlorite (included Chlorine in basic media), phosgene, phosphorous oxychloride, polyphosphates, alkaline-trimetaphosphates, polyfunctional silanes; epoxy-compounds, there derivatives and reactive oligo and polymers, like e.g. epichlorohydrin, derivatives of epichlorohydrin, e.g. mono- and multifunctional glycidyl ether, epoxy halides, substituted epoxides, polyepoxides; aliphatic dihalides, substituted polyethylene glycol, like e.g. diethylene glycol dichloride, triethylene glycol dichloride; Grafting reagents which are able to react further to cross-link, for example radical mediated cross-linking, polymerisation of double bonds, like e.g. acrylic acid derivatives, substituted acrylates, vinyl group containing compounds, aldehyde-amide-condensates; N,N,-dimethylol-imidzolidon-2 (DMEU), cyanuric chloride, biphenyl compounds, oxidised mono-, di- and oligosaccharides, any type boronate ester cross-linking.
And further variants of physically induced cross-linking: thermic process (water free), including melting, hydro thermic process (heat-water treatment), compounding, freeze-thaw-process. But the hydroxyalkyl starch or hydroxyalkyl starch fraction needs not to be cross-linked for the general purpose of the invention.

In one embodiment, the hydroxyalkyl starch or hydroxyalkyl starch fraction has at least one type of further substituents or modification. This means a substituent other than hydroxyalkyl, or a modification other than hydroxyalkylation. Additional substituents can adjust properties in agriculture use and cosmetics in case of viscosity, film forming properties, water solubility, gelling properties, water holding capacity, ion-binding, introduce charges, hydrophobicity, hydrophilicity. Useful substituents/modifications are: any kind types of esters, ethers, any alkyl (branched or not) group attached to the starch as ether with further functionalities or heteroatoms in the carbon chain (sulfur, oxygen, nitrogen, phosphorous, boron), like e.g. alkyl carboxylic acid, alkyl carboxylates, alkyl ester, alkyl ether, hydroxyalkyl, carbamoyl alkyl, oxidation of starch, reduction of starch, alkylamines, quaternary amines, quaternary alkylamines, alkyl amides, alkyl nitriles, carbamates, carbonates, alkylthiols, any types of inorganic groups, like e.g. phosphates, sulfates, silanes, silanol, siloxane, silyl ether, nitro groups, thiones, sulphides, sulfoxides, xanthates thiocarbamates, aldehydes, thioaldehydes, urea, guanidinium groups, carbonyl, alkyl carbonyl, alkyl halogenides, any type of grafting, for example radical grafting, like e.g. with acrylic acid derivatives, substituted acrylates, vinyl group containing compounds, aldehyde-amide-condensates. But the hydroxyalkyl starch or hydroxyalkyl starch fraction needs not to have a further type of substituent for the general purpose of the invention.

In a further aspect the invention provides a film, comprising a hydroxyalkyl starch or hydroxyalkyl starch fraction having an amylose content of at least 30 wt% and a weight average molar mass in the range of about 10⁵ g/mol to about 10⁸ g/mol, preferably in the range of about 10⁵ g/mol to about 4.10⁷ g/mol, and a salt which is selected from the group consisting of an alkaline metal salt and/or alkaline earth metal salt. Such film may be obtainable or obtained by at least partially drying a composition of the invention. Preferably, the composition is fully or substantially dried.

"Substantially dried" means a water content of about 15 wt% or less, or a water content of 5 wt% or less, more preferably 3 wt% or less, related to the weight of the dried composition of the film. The water content is preferably measured at a relative humidity of 50% at air temperature of 25°C.

With respect to ingredients of films or coating, which is mentioned hereinafter, we refer to above disclosure of a composition. Same ingredients as in the composition can be comprised in a film or a coating.

In a further aspect, the invention provides a seed, a soil, a plant, or a part of a plant, or an animal feedstuff, with a coating, wherein the coating comprises a hydroxyalkyl starch or a hydroxyalkyl starch fraction, having an amylose content of at least 30 wt% and a weight average molar mass in the range of about 10⁵ g/mol to about 10⁸ g/mol, preferably in the range of about 10⁵ g/mol to about 4.10⁷ g/mol, and a salt which is selected from the group consisting of an alkaline metal salt and/or alkaline earth metal salt, or the coating obtained or obtainable from a composition as described before, wherein the composition may be at least partially dried, or the coating being a film as described before.

The coating may be a film as mentioned above. The coating may be a film comprising or formed by the hydroxyalkyl starch or hydroxyalkyl starch fraction having an amylose content of at least 30 wt% and a weight average molar mass
in the range of about 10⁵ g/mol to about 10⁸ g/mol, preferably in the range of about 10⁵ g/mol to about 4.10⁷ g/mol. The coating comprises said salt which is selected from the group consisting of an alkaline metal salt and/or alkaline earth metal salt.

In one embodiment, the coating comprises an ingredient selected from an agrochemical, a biostimulator, or a plant strengthening agent, wherein said ingredient is comprised, particularly encapsulated, in the coating or adhered to the coating. Such coating may be obtained or obtainable from a formulation of the invention comprising said ingredient.

In a further aspect, the invention is directed to the use of a composition of the invention or a film of the invention, for coating of a seed, a soil, a growth medium, a plant, or a part of a plant, particularly coating of a surface of the plant or plant part, or an animal feedstuff. In other words, the invention provides a method of use of the composition or film of the invention wherein the composition or film of the invention is coated to a seed, a soil, a growth medium, a plant, or a part of a plant, particularly coated to a surface of the plant or plant part, or to an animal feedstuff.

The coating may mean a coating with the composition of the invention or with a partially or fully dried composition of the invention, or with a film of the invention. When a coating is a film of the invention such coating may be obtained from a composition of the invention which is at least partially dried after application to a surface or article to be coated. Coating, particularly of a seed, may be done after the treatment with an active substance or together with applying an active substance, such as an agrochemical.

The composition or film of the invention may be applied to a seed as a film forming binder. Another purpose of coating of a seed may be encrusting, pelleting for bonding, fixation of a substance selected from an agrochemical, an inoculant and a bio stimulant, on the seed surface, or improvement of germination. The composition or film of the invention may be used for covering seeds which are treated prior to covering with a substance, particularly with an agrochemical. All that similarly applies to a seed of the invention.

In seed coating in all forms such as film coating, encrusting and pelleting applications the formed starch films may improve the fixation and bonding of an applied active substance, such as an agrochemical, particularly a pesticide, a fertilizer, or a biostimulant on the seed surface. Dust formation, abrasion and spreading of such substance in the environment can be reduced by the composition or the film of the invention.

Seed coating with the composition, or the liquid formulation, also without further active substance or ingredient, improves the water holding capacity and germination.

The composition or film of the invention may be used for soil coating, particularly in order to reduce or prevent wind erosion or to reduce leaching, particularly of organic matter (preferably humus), clay silt or sand. Said substances may be bound or fixed to the soil by the composition of the invention or film of the invention. Soil coating may be done prior to treatment with an agrochemical. The composition or film of the invention may be used for soil bonding. The composition or film of the invention can for example be used to prevent leaching of an active substance, such as an agrochemical, particularly a fertilizer or a pesticide, from soil, particularly from a top soil. Soil coating may be done to improve water holding capacity of the soil. If the composition or film of the invention which is applied to a soil comprises an agrochemical, the applied agrochemical, particularly an applied pesticide, remains longer in a topsoil layer which favours the microbial biodegradation and reduces contamination of groundwater. All that similarly applies to a soil of the invention.

On sandy soils, addition of humic substances, like e.g. humic acid, fulvic acid, humates, as a part of the composition, will improve nutrient and mineral availability and uptake by plant roots, and water holding capacity (A. Noroozisharaf, M. Kaviani, Physiol Mol Biol Plants 24, 423-431 (2018).

A part of a plant (plant part) may for example be selected from leaves, blossom, flowers, stem, fruit, root, spike, awn, grain, ear, or roots.

The plant may be selected from crop plants, cereal, vegetables, fruits, nuts, fiber crops, such as cotton, spice plants, grasses, turf, wood providing plants, medical plants, ornamental plants, shrubs, garden plants.

The plant may specifically be selected from wheat, barley, rye, oat, corn (maize), rice, soy, canola, sugarcane, potato, sugar beet, manioc, water melon, banana, sweet potato, millet, onion, cucumber, lettuce, tomato, carrot, cabbage, Brussels sprouts, cauliflower, broccoli, kale, kohlrabi, cabbage, Chinese broccoli, collard greens, turnip, Chinese cabbage, napa cabbage, bok choy, radish, daikon, seedpod varieties, parsnip, beetroot, sea beet, Swiss chard, sugar beet, bean, eggplant, pumpkin, squash, marrow, zucchini (courgette), gourd, garlic, spinach, yam, cassava, apple, pear, cherry, orange, lemon, pineapple , coffee, tea, cacao, tobacco, peanuts, walnuts, olives, etc., ryegrass, alfalfa etc., lentils etc. roses and all other flowers etc. , .

Specific plants suitable for the invention are listed in the following articles, which are incorporated by reference:
https://de.wikipedia.org/wiki/Liste_von_Nutzpflanzen (version of January 14, 2020);
https://en.wikipedia.org/wiki/List of domesticated plants (version of January 14, 2020);
https://en.wikipedia.org/wiki/List of culinary fruits (version of January 14, 2020);
https://de.wikipedia.org/wiki/Liste der Obstarten (version of January 14, 2020);
https://de.wikipedia.org/wiki/Liste der Gemüse (version of January 14, 2020);
https://en.wikipedia.org/wiki/List of vegetables (version of January 14, 2020)
grasses, shrubs, trees/forestry, ornamentals, garden plants

The composition or film of the invention may be used as a leaf coating to reduce the wash-off from an active substance, such as an agrochemical, particular of a pesticide or a fertilizer, preferably a foliar fertilizer. Applying the aqueous formulation together with the active substance (such as plant protection products, e.g. fungicides, insecticides, bactericide and herbicides, or fertilizer, preferably foliar fertilizer) on leaves, reduces the wash-off due to a starch film formed after drying, reduces unwanted wash-off and the active substance (also called active ingredient) remains in its place of action, and premature contamination of soil is avoided. Therefore, the invention reduces unwanted soil and groundwater contamination by wash-off of the active substance, particularly pesticides or fertilizers, which were applied on a plant, particularly on leaves.

Root coating may have the effect of, or be done for, improved moisturizing including water availability, water use efficiency and water uptake. The formulation or film of the invention can be applied on roots to improve the water holding capacity, root growth, storage stability, improved nutrient uptake and/or to improve the fixation and bonding of an applied agrochemical, an applied biostimulant, an applied microorganism and/or an applied root growth promoting substance on the root surface.

Coating of fruits and vegetables may have the effect, or be done, to avoid osmotic bursting (food cracking) during rainfall. Therefore, the composition or film of the invention can be used as protecting film to reduce bursting of fruits and vegetables due to increased osmotic pressure in the occasion of rainfall (food cracking).

The composition or film of the invention may be used as a coating of spikes or ears, particularly in monocotyledonous plants, such as in grasses and in many other plants that preferably show severe seed shattering. This has the effect, that loss of seeds before harvest, due to impact of wind and rain, can be reduced or avoided.

In this regard, the composition or film of the invention may be used as binder of spikes, ears and seeds.

The composition or film of the invention may be applied on animal feedstuff as a binder. It is natural, non-hazardous and edible. Here, the composition or film of the invention may also be used to improve abrasion resistance and therefore dust reduction. The present invention can reduce the exposure to dust from raw materials of animal food, since almost all products used in the animal feed industry are in powder form. Many of them are chemical compounds and biological agents have both, acute and long-term toxic effects like e.g. skin and respiratory sensitisation.

In a further aspect, the invention is directed to the use of a composition of the invention or a film of the invention as a carrier matrix for an agrochemical, a biostimulator or a plant strengthening agent, or as a biostimulant. In other words, the invention provides a method of use of the composition wherein an agrochemical, a biostimulator and/or a plant strengthening agent is put into the composition which forms a carrier matrix for one or more of these, or a method of use of the composition or film of the invention, itself, as biostimulant.

The composition or the film of the invention can be used as carrier matrix for an active substance, for example an agrochemical, particularly a plant protection product, e.g. a pesticide, fungicide, insecticide, bactericide, herbicide, or for a fertilizer. The active substance can be embedded in the composition, or in an at least partially dried composition, or in the film of the invention. The composition, the at least partially dried composition or the film can act as a slow-release matrix. In the composition or film, active substances, like agrochemicals or plant strengthening agents can be included and released. The composition or film enables delayed transport, delayed diffusion, controlled release and slow release. This results in reduced plant stress caused by the active substances or co-formulants which contain plant protection products. For example, a higher pesticide efficiency by slow release and long-term activity reduces plant stress. This also applies to embodiments of the invention where the composition or film of the invention is used as a coating.

The composition of the invention or the film of the invention may comprise a biostimulator, as a further substance, or the composition of the invention or the film of the invention may itself be a biostimulant. The composition of the invention or the film of the invention can be used as biostimulant for plants to reduce the impact of external stress like drought, solar radiation, frost and rainfall. The composition of the invention or the film of the invention may be applied on soil to ensure the stimulation of microbial activity. This can particularly be done when the composition of the invention or the film of the invention is used as a coating. The composition of the invention or the film of the invention may be used to form a biostimulant film on leaves to reduce the impact of external stress like drought, solar irradiation, frost and rainfall and for stimulation of microbial activity in the soil.

In a further aspect, the invention is directed to the use of a composition of the invention or a film of the invention as a dust binding agent or dust preventing agent. In other words, the invention provides a method of use of the composition of the invention or the film of the invention for reducing dust. The composition of the invention or the film of the invention may particularly be used to reduce dust in animal production facilities, wherein the animal production facilities are feed lots, pasture, stables, barns and horse racetracks. The composition of the invention or the film of the invention may be applied onto a surface or onto an object, or brought into an environment, for example by spraying.

In the uses of the invention or the method of uses of the invention, the composition may be applied, for example onto seed, a soil, a plant, or a part of a plant, or an animal feedstuff, and at least partially dried, preferably substantially dried. Thereby, a film of the invention can be formed. "Substantially dried" means a water content of about 15 wt% or less, or a water content of 5 wt% or less, more preferably 3 wt% or less, related to the weight of the dried composition of the film. The water content is preferably measured at a relative humidity of 50% at air temperature of 25°C.

In a further aspect, the invention is related to the use of a composition as described before or of a film as described herein, as, or for, a cosmetic or personal care application. The formulation may particularly be used as thickening agent, film forming agent, moisturizing agent, barrier forming agent, wetting agent, sticking agent, gelling agent, protective barrier and/or rheological additive.

The invention also provides a cosmetic comprising a composition as described before or of a film as described before.

The invention also provides a personal care application comprising a composition as described before or of a film as described before.

The invention also discloses a cosmetic, comprising
- a hydroxyalkyl starch or a hydroxyalkyl starch fraction, having an amylose content of at least 30 wt% and a weight average molar mass in the range of about 10⁵ g/mol to about 10⁸ g/mol, preferably in the range of about 10⁵ g/mol to about 4.10⁷ g/mol,
- a salt which is selected from the group consisting of an alkaline metal salt and/or alkaline earth metal salt.

The cosmetic may comprise the hydroxyalkyl starch or hydroxyalkyl starch fraction in an amount of 0.001 to 10wt%, based on the whole mass of the cosmetic, in an amount of 0.001 to 5 wt%, in an amount of 0.001 to 2 wt% or in an amount of 0.001 to 1 wt%.

The cosmetic may comprise the salt in an amount of 0.001 to 1 wt%, based on the whole mass of the cosmetic, in an amount of 0.001 to 0.5 wt%, in an amount of 0.001 to 0.2 wt% or in an amount of 0.001 to 0.1 wt%.

The cosmetic may comprise the composition of the invention uniformly distributed, e.g. in bulk, or concentrated at a location.

The cosmetic may comprise the film of the invention as a coating.

Preferable cosmetics or personal care applications are selected from cremes, sprays, lotion, shampoo, shaving cream, shaving gel, hair care and hair styling products, skin care and color cosmetics, sun screen , lip stick, lip balms, soap, lotion, hair conditioner, hair straightener, makeup, nailcare products, toothpaste, oral hygiene and care products.

Due to the viscoelastic character, the liquid formulation can be used to increase the viscosity of cosmetic and personal care products or improve the stability of oily emulsions. The viscosity in the final product can be adjusted by parameters including starch content, degree of substitution and average molecular weight of the used starch. As further aspect, the film forming properties makes the liquid formulation applicable as film former in cosmetic and personal care products like hair stylings, skin care products and color cosmetics. Film forming substances, currently mostly acrylates, form a transdermal film on skin to avoid skin dehydration, as protection against external influences and as styling holder in hair products. Nowadays, substitutions for environmental problematic synthetic substances such as acrylates are in high demand. Additionally, the water holding capacity of the liquid starch formulation can be used to add a moisturizing and wetting effect in skin care products.

### BRIEF DESCRIPTION OF THE FIGURES

- Fig. 1:: Molar mass determination of the starch containing the composition of the invention by SEC-MALLS.
- Fig. 2:: Determination of the degree of substitution (DS) and molar substitution (MS) by high resolution ¹³C-NMR in D₂O. ;
- Fig. 3a,b:: Flow curves of two independent starch-based compositions (formulations, invention), each after 1 day and 6 months;
- Fig. 4:: Frequency sweeps of the starch formulation;
- Fig. 5:: Determination of the degree of substitution (DS) by high resolution ¹³C-NMR in D₂O.
- Fig. 6:: shows a bar graph showing mancozeb residues remaining on foliage after spray application of formulations comprising mancozeb in combination with either the invented starch formulation as adjuvant formulation or as fungicide alone, 6 days after application and 27 mm of rainfall in 2009 (A) and 4 days after application and 24 mm of rainfall in 2010 (B).
- Fig. 7:: shows a bar graph from fluazinam residues remaining on foliage after spray application of formulations comprising fluazinam in combination with either the composition of the invention as adjuvant formulation or as fungicide alone, 6 days after application and 27mm of rainfall in 2009 (A) and 4 days after application and 24mm of rainfall in 2010 (B);
- Fig. 8:: shows a bar graph referring to dimethomorph residues remaining on foliage after spray application of formulations comprising dimethomorph in combination with either the invented starch formulation adjuvant formulation or as fungicide alone, 6 days after application and 27mm of rainfall in 2009 (A) and 4 days after application and 24mm of rainfall in 2010 (B).
- Fig. 9:: Influence of a starch-based formulation on the reduction of stress, caused by fungicide application under hot and dry weather conditions in 2018.

### EXAMPLES

### Methods:

The weight average molar mass of the starch polymer was determined by SEC-MALLS as follows: The HPSEC system consisted of a 600MS pump module, a 717 autoinjector, column compartment, a RI-detector 410, and a MALLS detector Dawn-F-DSP laser photometer (Wyatt Technology, Santa Barbara). The three columns used were suprema of company PSS: 10⁸ - 10⁶ g/mol; 2·10⁶ - 5·10⁴ g/mol; 10³ - 10⁵ g/mol. They had dimensions of 300 · 7.8 mm. Elution of the samples was carried out with H₂O containing 0.05 m NaNO₃ at a flow rate of 0.735 mL·min⁻¹ and a temperature of 40°C. The concentrations ranged from 1 to 5 mg·mL⁻¹ depending on the expected molar mass. The MALLS detector was serially connected with the refractive index detector (DRI).

The degree of substitution (DS) and the molar degree of substitution (MS) was determined by ¹³C-NMR spectroscopy of the hydroxypropyl starch after total hydrolysis. The DS value was determined from the mean value of the signals of C2-, C3- and C6-position in relation to the signal in C1-position as described by J. Kunze, A. Ebert, H.-P. Fink, Cellul. Chem. Technol. 2000, 34, 21-34.

The degree of substitution (DS) and the molar degree of substitution (MS) was determined by ¹³C-NMR spectroscopy of the hydroxypropyl starch after total hydrolysis. The DS value was determined by integration of the methyl group signals of hydroxypropyl groups directly attached to the C2-, C3- and C6-position and for the MS determination the additional hydroxypropyl groups attached to the hydroxy group in the chain of the hydroxypropyl substituent in relation to the signals for the C1-position as described by J. Kunze, A. Ebert, H.-P. Fink, Cellul. Chem. Technol. 2000, 34, 21-34.

The amylose content was determined amperometric, refering to the method described by Richter, Augustat and Schierbaum (Ausgewahlte Methoden der Starkechemie. Wiss. Verlagsgesellschaft mbH. Stuttgart (1968). 111).

The morphological analysis (e.g. amorphous, crystalline, particular) was carried out by light microscopy with polarized light.

All rheological measurements where applied at constant 25°C on a Physica Rheolab MC 100.

### Working examples:

### 1. Preparation of formulations

### EXAMPLE 1: MANUFACTURING PROCEDURE FOR A LIQUID FORMULATION FOR AGRICULTURE, HORTICULTURE AND FORESTRY, AND ANIMAL HUSBANDARY.

High amylose starch containing 68% amylose was gelatinised under alkaline conditions (pH = 12-14) without any swelling inhibitor and permanent stirring in the temperature range between 20-60 °C for 2-8 hours until the starch granules were completely disrupted and dispersed (transparent dispersion). Afterwards, the starch paste was hydroxypropylated according the MS in the range of 0.1 to 0.8 with propylene oxide. The resulting degree of substitution of the derivative was DS = 0.17 and the molar degree of substitution was MS = 0.35. For the solution, the pH was adjusted to 6.2 and additional humic acid and geraniol were added. The final concentrations of the components in the aqueous formulation were: 10 wt% starch, 8 wt% potassium phosphate, 0.01 wt% humic acid and 0.1 wt% of geraniol.

### EXAMPLE 2: MANUFACTURING PROCEDURE FOR A LIQUID FORMULATION IN COSMETICS AND PERSONAL CARE

Manufacturing procedure for a liquid formulation in cosmetic and personal care products: High amylose starch containing 68% amylose was gelatinised under alkaline conditions (pH = 12-14) without any swelling inhibitor and permanent stirring in the temperature range between 20-60 °C for 2-8 hours until the starch granules were completely disrupted and dispersed (transparent dispersion). Afterwards, the starch paste was hydroxypropylated according the MS in the range of 0.1 to 0.8 with propylene oxide. For the aqueous solution, the pH was adjusted to 6.2 and if necessary, desalinated until the conductivity reached a value ≤ 1500 µS/cm. The final concentrations of the components in the aqueous formulation were: 10 wt% starch.

### EXAMPLE 3: ANALYTICAL DATA OF THE STARCH BASED FORMULATION

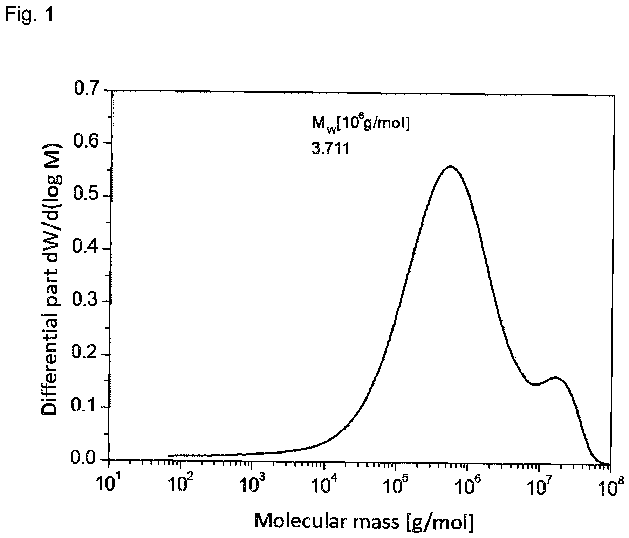

### Determination of molecular mass (Fig. 01)

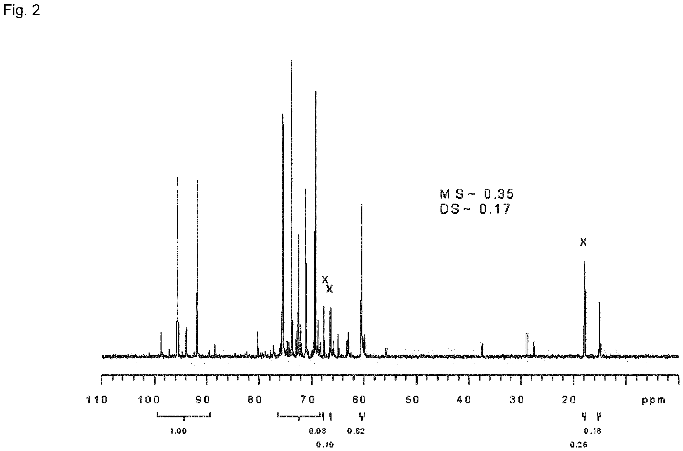

### Determination of DS and MS by ¹³C-NMR in D₂O (Fig. 02)

To obtain higher resolution the starch containing formulation (invention) was first hydrolyzed with trifluoracetic acid.

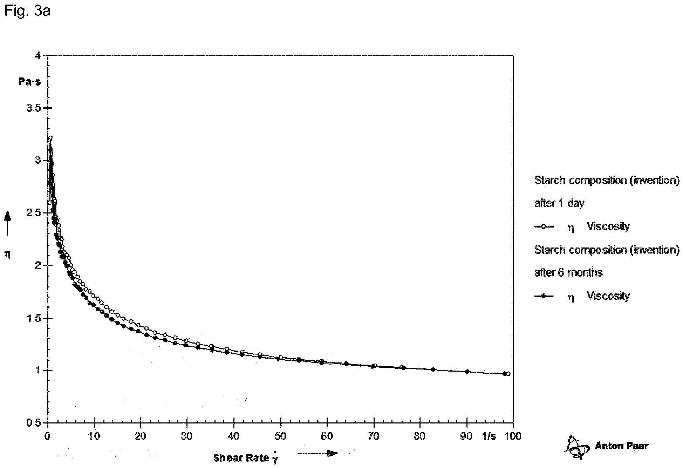

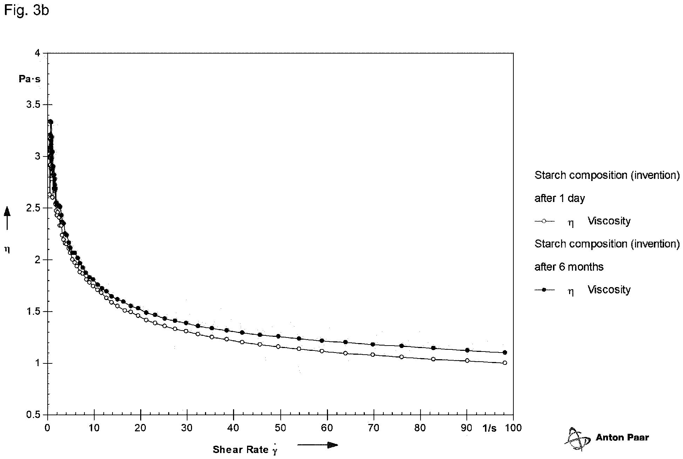

### Flow curves of the Invention (Fig. 03)

In consequence, the starch composition shows long-term stable viscosity.

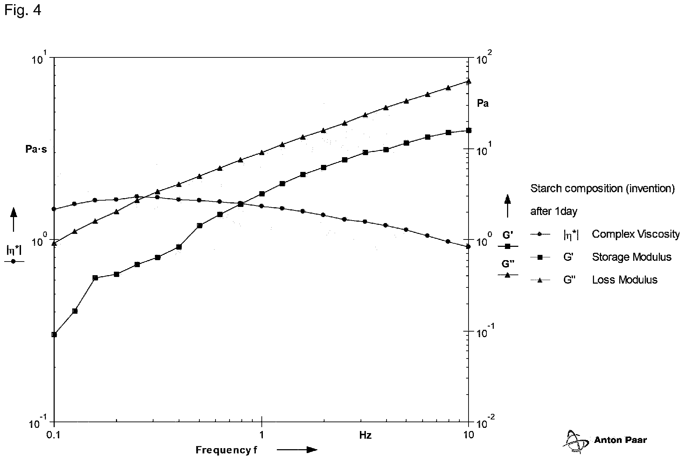

### Frequency sweep of the Invention (Fig. 04)

Loss and storage modulus are strongly dependent on the frequency at deformation of the viscoelastic range and G" > G' in the frequency range from 0.1 - 10 Hz, show a liquid state.

**Table 1: Results of amperometric amylose determination.**

| **Sample** | **JBV [%]** | **Amylose [%]** | **Average/RSD [%]** | **[%]** |
|---|---|---|---|---|
| **Sample 1** | 13.95 | 68.06 | 67.86 | 67.9 |
| | 13.87 | 67.66 | 0.2 | 0.2 |
| | 13.91 | 67.86 | | |
| | 13.94 | 68.00 | 68.05 | |
| | 13.97 | 68.14 | 0.1 | |
| | 13.94 | 68.00 | | |
| | 13.91 | 67.84 | 67.87 | |
| | 13.94 | 68.01 | 0.1 | |
| | 13.89 | 67.77 | | |

### EXAMPLE 4: DETERMINATION OF THE SUBSTITUENT DISTRIBUTION FROM THE STARCH BASED FORMULATION

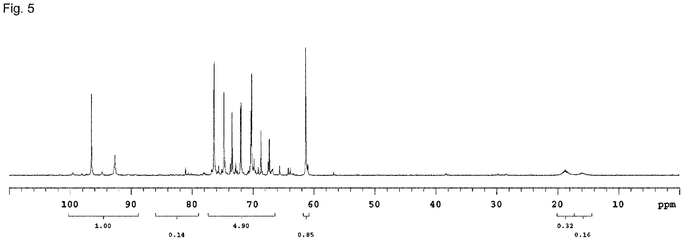

The degree of substitution (DS) of a modified hydroxypropyl starch was determined by ¹³C-NMR spectroscopy after total hydrolysis of the starch ether as described before. The found DS value was 0.30 (Figure 05).
The proportion of unsubstituted glucose units was determined from the same completely hydrolysed sample by HPAE-PAD using calibration with glucose standards. The result was 0.75.
According to Spurlin (H.M. Spurlin Journal of the American Chemical Society 1939, 61, 2222-2227), assuming the same reaction rates for the three hydroxyl groups, the proportion of unsubstituted glucose units is 0.73 at a DS of 0.30. The small difference between the experimentally determined value and the theoretically calculated value indicates that the product is substituted largely homogeneous.

### EXAMPLE 5: ACTIVE INGREDIENT REMAINING ON FOLIAGE AFTER RAINFALL

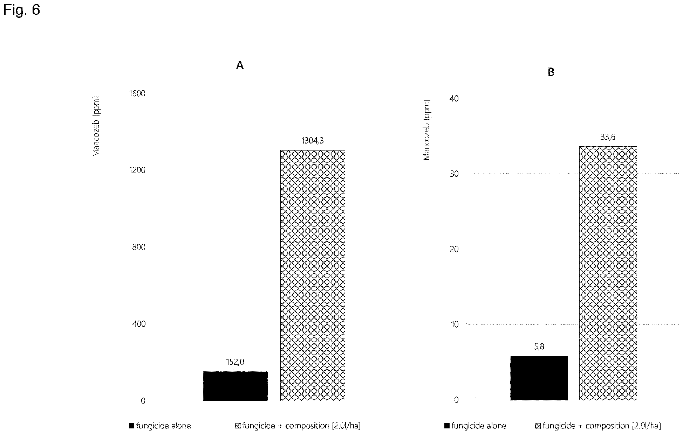

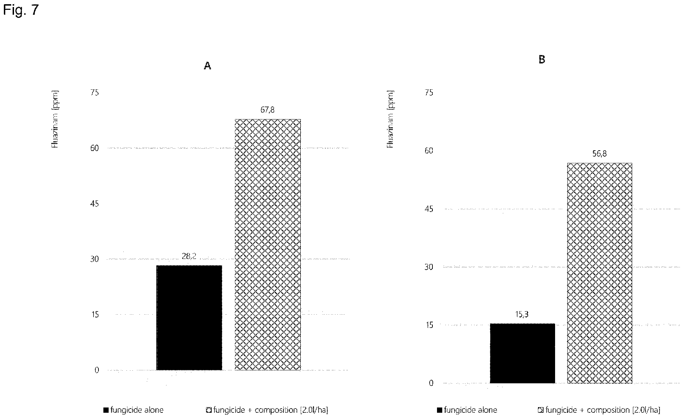

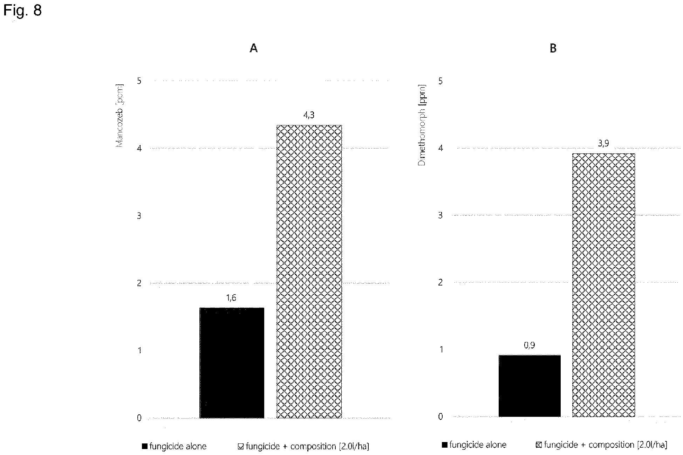

Improved rain fastness of Mancozeb (Fig. 06), fluazinam (Fig. 07) and dimethomorph (Fig. 08) on leaves by the invention.

### EXAMPLE 6: ACTIVE HERBICIDE INGREDIENT REDUCTION

Comparison of the efficacy of standard herbicide application without any adjuvant and with a fifty percent (50%) reduced herbicide application in combination with the starch-based formulation (invention) which was tested in GEP-trials (Good Experimental Practices) in three 3 consecutive years.

**Table 2: The following herbicide formulations were used:**

| Sample Nr. | name | type^{a} | rate | unit |
|---|---|---|---|---|
| 1 | Bandur | SC | 4,0 | l/ha |
| | Sencor WG | WG | 0,5 | kg/ha |
| 2 | Bandur | SC | 2,0 | l/ha |
| | Sencor WG | WG | 0,25 | kg/ha |
| | Starch based formulation | SC | 6,0 | l/ha |

| | | | | |
|---|---|---|---|---|
| ^{a}SC = suspension concentrate, WG = water dispersible granules | | | | |

**Table 3: Herbicide efficacy of an inventive application consisting of a mixture of herbicides and a starch-based formulation in standard and reduced application rate. In 2014, 24 days after application.**

| weed | Herbicides: 100 % | Herbicides 50% + formulation |
|---|---|---|
| | Herbicide efficacy [%] | |
| *Elymus repens* [AGRRE] | 98 | 99 |
| *Viola arvensis* [VIOAR] | 100 | 100 |
| *Polygonum aviculare* [POLAV] | 97 | 100 |
| *Geranium dissectum* [GERDI] | 99 | 100 |
| *Brassica napus* [BRSNN] | 100 | 100 |
| *Matricaria chamomilla* [MATCH] | 100 | 100 |
| *Fallopia convolvulus* [POLCO] | 99 | 100 |

**Table 4: Herbicide efficacy of an inventive application consisting of a mixture of herbicides and a starch-based formulation in standard and reduced application rate. In 2014, 54 days after application.**

| weed | Herbicides: 100 % | Herbicides 50% + formulation |
|---|---|---|
| | Herbicide efficacy [%] | |
| *Elymus repens* [AGRRE] | 91 | 95 |
| *Viola arvensis* [VIOAR] | 100 | 100 |
| *Polygonum aviculare* [POLAV] | 96 | 100 |
| *Geranium dissectum* [GERDI] | 100 | 100 |
| *Brassica napus* [BRSNN] | 100 | 100 |
| *Matricaria chamomilla* [MATCH] | 100 | 100 |
| *Fallopia convolvulus* [POLCO] | 97 | 100 |

**Table 5: Herbicide efficacy of an inventive application consisting of a mixture of herbicides and a starch-based formulation (invention) in standard and reduced application rate. In 2014, 67 days after application.**

| weed | Herbicides: 100 % | Herbicides 50% + formulation |
|---|---|---|
| | Herbicide efficacy [%] | |
| *Elymus repens* [AGRRE] | 86 | 95 |
| *Viola arvensis* [VIOAR] | 100 | 100 |
| *Polygonum aviculare* [POLAV] | 94 | 100 |
| *Geranium dissectum* [GERDI] | 100 | 100 |
| *Brassica napus* [BRSNN] | 100 | 100 |
| *Matricaria chamomilla* [MATCH] | 100 | 100 |
| *Fallopia convolvulus* [POLCO] | 96 | 100 |

**Table 6: Herbicide efficacy of an inventive application consisting of a mixture of herbicides and a starch-based formulation (invention) in standard and reduced application rate. In 2015, 24 days after application.**

| weed | Herbicides: 100 % | Herbicides 50% + formulation |
|---|---|---|
| | Herbicide efficacy [%] | |
| *Elymus repens* [AGRRE] | 69 | 75 |
| *Sonchus arvensis* [SONAR] | 49 | 65 |
| *Convolvulus arvensis* [CONAR] | 99 | 100 |
| *Equisetum arvense* [EQUAR] | 59 | 49 |
| *Vicia cracca* [VICCR] | 99 | 100 |
| *Viola arvensis* [VIOAR] | 100 | 100 |
| *Chenopodium album* [CHEAL] | 100 | 100 |
| *Matricaria chamomilla* [MATCH] | 100 | 100 |
| *Fallopia convolvulus* [POLCO] | 98 | 100 |

**Table 7: Herbicide efficacy of an inventive application consisting of a mixture of herbicides and a starch-based formulation (invention) in standard and reduced application rate. In 2016, 18 days after application.**

| weed | Herbicides: 100 % | Herbicides 50% + formulation |
|---|---|---|
| | Herbicide efficacy [%] | |
| *Poa annua* [POAAN] | 89 | 93 |
| *Geranium dissectum* [GERDI] | 100 | 100 |
| *Cirsium arvense* [CIRAR] | 100 | 100 |
| *Vicia cracca* [VICCR] | 100 | 100 |
| *Viola arvensis* [VIOAR] | 100 | 100 |
| *Fallopia convolvulus* [POLCO] | 96 | 100 |

### EXAMPLE 7: STRESS REDUCTION

Table 8: Comparison of the efficacy of standard fungicide application, with a fifty percent (50%) reduced fungicide application, both in combination with and without a starch-based formulation (invention) was tested in a GEP-trial. Infestation of potatoes with *Phytophthora infestans* (PHYTIN) was low (under 1%) in all treatments, due to very dry and hot weather conditions. The influence of the addition of the starch-based formulation (invention) on stress caused by fungicide treatments was evaluated. A positive effect on potato yield is shown, due to fungicide-stress reduction.

**The following fungicide treatments were evaluated:**

| Sample Nr. | name | Type | Rate | unit |
|---|---|---|---|---|
| 1 | Ridomil Gold MZ | WG | 2.0 | kg/ha |
| | Acrobat Plus WG | WG | 2.0 | kg/ha |
| | Shirlan | SC | 0.4 | l/ha |
| | Ranman Top | SC | 0.5 | l/ha |
| 2 | Ridomil Gold MZ | WG | 2.0 | kg/ha |
| | Acrobat Plus WG | WG | 2.0 | kg/ha |
| | Shirlan | SC | 0.4 | l/ha |
| | Ranman Top | SC | 0.5 | l/ha |
| | Starch based formulation | SC | 2.0 | l/ha |
| 3 | Ridomil Gold MZ | WG | 2.0 | kg/ha |
| | Acrobat Plus WG | WG | 2.0 | kg/ha |
| | Shirlan | SC | 0.4 | l/ha |
| | Ranman Top | SC | 0.5 | l/ha |
| 4 | Ridomil Gold MZ | WG | 2.0 | kg/ha |
| | Acrobat Plus WG | WG | 2.0 | kg/ha |
| | Shirlan | SC | 0.4 | l/ha |
| | Ranman Top | SC | 0.5 | l/ha |
| | Starch based formulation | SC | 2.0 | l/ha |

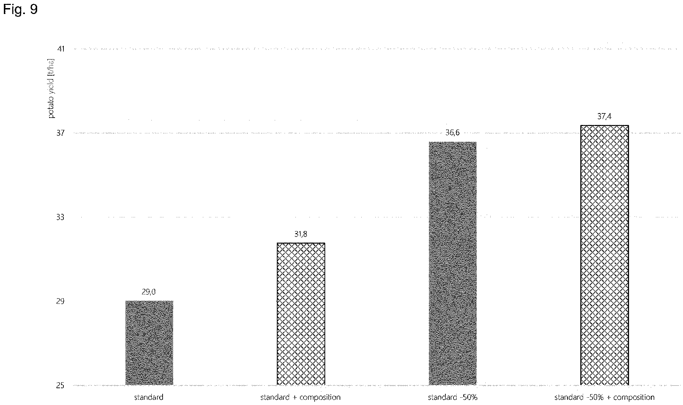

Influence of a starch-based formulation on the reduction of stress (Fig. 09)

## Claims

1. A composition comprising
- a hydroxyalkyl starch or a hydroxyalkyl starch fraction, having an amylose content of at least 30 wt% and a weight average molar mass in the range of about 10⁵ g/mol to about 10⁸ g/mol,
- a salt which is selected from the group consisting of an alkaline metal salt and/or alkaline earth metal salt,
- water.

2. The composition of claim 1, wherein the content of the hydroxyalkyl starch or the hydroxyalkyl starch fraction is about 5 wt% to about 25 wt%.

3. The composition of any of the preceding claims, wherein the content of the salt is 0.01 wt% to about 10 wt%.

4. The composition of any of the preceding claims, wherein the hydroxyalkyl starch or the hydroxyalkyl starch fraction has a degree of molar substitution (MS) from about 0.05 to about 0.8.

5. The composition of any of the preceding claims, wherein the hydroxyalkyl starch or the hydroxyalkyl starch fraction has a predominantly non-granular, amorphous structure.

6. The composition of any of the preceding claims, wherein the hydroxyalkyl starch or the hydroxyalkyl starch fraction is predominantly dissolved in the water.

7. The composition of any of the preceding claims, wherein the salt has a chalcogen or pnictogen comprising anion.

8. The composition claim 7, wherein the anion is selected from phosphate, hydrogen phosphate, sulfate, hydrogen sulfate or acetate.

9. The composition of any of the preceding claims, further comprising an ingredient selected from the group consisting of terpenes, a humic substance, an agrochemical, a biostimulator, a plant strengthening agent, or a preserving substance.

10. The composition of any of the preceding claims, wherein the hydroxyalkyl starch or the hydroxyalkyl starch fraction has a degree of substitution (DS) from about 0.025 to about 0.6.

11. A film comprising a hydroxyalkyl starch or a hydroxyalkyl starch fraction having an amylose content of at least 30 wt% and a weight average molar mass in the range of about 10⁵ g/mol to about 10⁸ g/mol, and a salt which is selected from the group consisting of an alkaline metal salt and/or alkaline earth metal salt, or the film obtained or obtainable by at least partially drying a composition of one of claims 1-10.

12. A seed, a soil, a plant, or a part of a plant, or an animal feedstuff, comprising a coating, the coating comprising a hydroxyalkyl starch or a hydroxyalkyl starch fraction having an amylose content of at least 30 wt% and a weight average molar mass in the range of about 10⁵ g/mol to about 10⁸ g/mol and a salt which is selected from the group consisting of an alkaline metal salt and/or alkaline earth metal salt, or the coating obtained or obtainable from a composition of any of claims 1 - 10, wherein the composition may be at least partially dried, or the coating being a film of claim 11.

13. The seed, soil, plant, or part of a plant according to claim 12, wherein the coating comprises an ingredient selected from the group consisting of an agrochemical, a biostimulator, or a plant strengthening agent, wherein said ingredient is comprised in the coating or adhered to the coating.

14. Use of a composition of one of claims 1-10, or of a film of claim 11, for coating of a seed, a soil, a growth medium, a plant, or a part of a plant, or an animal feedstuff.

15. Use of a composition of one of claims 1-10, or of a film of claim 11, as
- a carrier matrix for an ingredient selected from an agrochemical, a biostimulator or a plant strengthening agent,
- a biostimulant,
- a dust binding agent or
- a dust preventing agent.

16. Use of a composition of one of claims 1-10, or of a film of claim 11, as, or for, a cosmetic or personal care application.

17. The use of claim 16, wherein the composition is used as thickening agent, film forming agent, moisturing agent, barrier forming agent, wetting agent, sticking agent, gelling agent, protective barrier and/or rheological additive.

18. A cosmetic or a personal care application, comprising a composition of one of claims 1-10, or of a film of claim 11.
